# EUROPÄISCHE PATENTANMELDUNG

(11) **EP 0 539 821 A2**
(43) Veröffentlichungstag der Anmeldung: **05.05.1993**
(21) Anmeldenummer: 92117791.1
(22) Anmeldetag: 19.10.1992
(51) Int. Cl.: C07C 213/04, C10M 133/08, C10M 133/54, C10L 1/22, C07C 215/08, C07D 295/08

(54) **Verfahren zur Herstellung von Alkanolaminen und Verwendung des Reaktionsprodukts als Kraft- oder Schmierstoffadditiv**

(30) Priorität: 31.10.1991 DE 4135946
(71) Anmelder: BASF Aktiengesellschaft, D-67063 Ludwigshafen (DE)
(72) Erfinder: Dingerdissen, Uwe, Dr., W-6104 Seeheim-Jugenheim 1 (DE); Hoelderich, Wolfgang, Prof. Dr., W-6710 Frankenthal (DE); Mohr, Juergen, Dr., W-6718 Gruenstadt (DE); Oppenlaender, Knut, Dr., W-6700 Ludwigshafen (DE); Thomas, Juergen, Dr., W-6701 Fussgoenheim (DE)

(57) **Zusammenfassung**

Verfahren zur Herstellung von Alkanolaminen aus Epoxiden und Aminen unter Verwendung von Feststoffkatalysatoren und Verwendung des Reaktionsprodukts als Kraftstoff- oder Schmierstoffadditiv

## Beschreibung

Die vorliegende Erfindung betrifft ein Verfahren zur Herstellung von Alkanolaminen aus Epoxiden und die Verwendung des Reaktionsprodukts als Kraftstoff- und Schmierstoffadditiv. Der Zusatz dieser Alkanolamine zu Kraftstoffen für Verbrennungsmotoren und Schmierstoffen bewirkt eine Reinhaltung von Ventilen und Vergasern.

Vergaser und Einlaßsystem von Ottomotoren, aber auch Einspritzsysteme für die Kraftstoffdosierung in Otto- und Dieselmotoren, werden in zunehmendem Maße durch Verunreinigungen belastet, die durch Staubteilchen aus der Luft, unverbrannte Kohlenwasserstoffreste aus dem Brennraum und die in den Vergaser geleiteten Kurbelwellengehäuseentlüftungsgase verursacht werden.

Die Rückstände verschieben das Luft-Kraftstoffverhältnis im Leerlauf und im unteren Teillastbereich, so daß das Gemisch fetter, die Verbrennung unvollständiger und wiederum die Anteile unverbrannter oder teilverbrannter Kohlenwasserstoffe im Abgase größer werden und der Benzinverbrauch steigt.

Es ist bekannt, daß zur Vermeidung dieser Nachteile Kraftstoffadditive zur Reinhaltung von Ventilen und Vergaser bzw. Einspritzsystemen verwendet werden (vgl. z.B.: M. Rossenbeck in Katalysatoren, Tenside, Mineralöladditive, Hrsg. J. Falbe, U. Hasserodt, S. 223 f., G. Thieme Verlag, Stuttgart, 1978).

Je nach Wirkungsweise, aber auch nach dem bevorzugten Wirkort solcher Detergents-Additive unterscheidet man heute zwei Generationen.

Die erste Additiv-Generation konnte nur die Bildung von Ablagerungen im Ansaugsystem verhindern, nicht aber bereits vorhandene Ablagerungen wieder entfernen, wohingegegen die Additive der zweiten Generation beides bewirken können ("keep-clean-" und "clean-up-Effekt") und zwar aufgrund ihrer hervorragenden Thermostabilität, insbesondere auch an Zonen höherer Temperaturen, nämlich an den Einlaßventilen.

Das molekulare Bauprinzip von Kraftstoff-Detergentien kann verallgemeinernd angegeben werden als Verknüpfung polarer Strukturen mit meist höhermolekularen, unpolaren oder lipophilen Resten.

Vertreter der zweiten Additiv-Generation sind oft Produkte auf der Basis von Polyisobutenen im unpolaren Molekülteil. Hier wiederum sind Additive vom Polyisobutylamin-Typ besonders hervorzuheben. Polyisobutylamine erhält man, ausgehend von Polyisobutenen, im wesentlichen nach zwei Verfahren. Das erste verläuft über eine Chlorierung des polymeren Grundkörpers und anschließenden nukleophilen Ersatz durch Amino oder vorzugsweise Ammoniak. Der Nachteil dieses Verfahrens ist die Verwendung von Chlor und das Auftreten von Chlor- oder Chlorid-haltigen Produkten, die keinesfalls mehr erwünscht sind und soweit irgend möglich gemieden werden (DE-OS 21 29 461, DE-OS 22 45 918).

Im zweiten Verfahren wird ein reaktives Polyisobuten zunächst in einer Oxosynthese carbonyliert und danach in Anwesenheit von Ammoniak reduktiv aminiert (DE-OS 36 11 230).

Günstigere Eigenschaften erzielt man durch den Einsatz von Alkanolaminen.

Aus der nichtvorveröffentlichten DE 40 30 164 sind Polyisobutylaminoalkohole bekannt, die durch Epoxidierung der entsprechenden Polyisobutene und anschließend durch Umsetzung mit Ammoniak oder Aminen erhalten werden. Die Synthese des Epoxids gelingt mittels bekannter Epoxidierungsmittel (Peressigsäure, m-Chlorperbenzoesäure, Hydroperoxiden).

Unter den Bedingungen der dort beschriebenen homogenen Katalyse läßt sich in Gegenwart von H₂O die Bildung von Diolen durch Hydrolyse der Epoxide nicht ganz vermeiden. Zudem sind die Ausbeuten noch nicht vollständig befriedigend.

Der Einsatz von chlorhaltigen Lewissäuren als Homogenkatalysator, wie in der DE-OS 23 31 290 beschrieben, hat ebenso erhebliche Nachteile. Die Katalysatoren haben korrosive Eigenschaften, ihre Aufarbeitung muß hydrolytisch erfolgen und die daraus entstehenden Salze müssen umweltgerecht entsorgt werden. Zusätzlich entstehen chlorierte Kohlenwasserstoffe als Nebenprodukte.

Der Erfindung lag daher die Aufgabe zugrunde, ein verbessertes Verfahren zur Herstellung von Alkanolaminen in hoher Reinheit zur Verfügung zu stellen.

Überraschenderweise werden die oben aufgeführten Nachteile vermieden durch das erfindungsgemäße Verfahren zur Herstellung von Alkanolaminen der Formel I 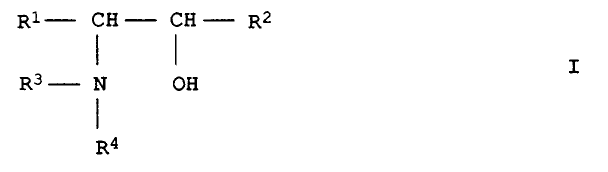
worin bedeuten
R¹ und R² unabhängig voneinander Wasserstoff oder einen, gegebenenfalls Aryl-substituierten, gesättigten oder ungesättigten aliphatischen Rest mit 25 bis 350 C-Atomen, mit der Maßgabe, daß zumindest einer der beiden Reste R¹ und R² der genannte Alkylrest ist und die Summe der C-Atome in den Resten R¹ und R² 25 bis 350 beträgt,
und
R³ eine OH- Gruppe
sowie
R³ und R⁴ unabhängig voneinander Wasserstoff oder Alkyl-, Hydroxyalkyl-, Aryl-, Aralkyl-, Alkaryl- oder Aminoalkylreste, die noch durch weitere Hydroxy- oder Aminogruppen tragende Alkylreste substituiert sein können, wobei R³ und R⁴ zusammen einen heterocyclischen Ring bilden können,
das dadurch gekennzeichnet ist, daß man Expoxide der Formel II 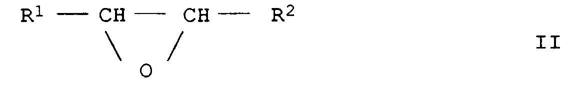
in Gegenwart von Feststoffkatalysatoren mit NH₃ oder Aminen der Formel III 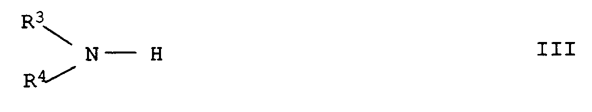
umsetzt, wobei R¹, R², R³ und R⁴ die oben genannten Bedeutungen aufweisen.

Bevorzugt bedeuten
R³ und R⁴ unabhängig voneinander Wasserstoff, einen C₁-C₁₀-Alkylrest, einen C₆-C₁₀-Arylrest, einen C₇-C₂₀-Aralkylrest, einen C₁-C₈-Hydroxyalkylrest, einen C₇-C₂₀-Alkylarylrest oder einen Aminoalkylrest der Formel IV

⁅R⁵―NR⁶⁆ₘR⁷ IV

worin R⁵ für einen C₂-C₅-Alkylenrest und R⁶ sowie R⁷, die gleich oder verschieden sind, für Wasserstoff, einen C₁-C₆-Alkylrest, C₆-C₁₀-Arylrest oder C₁-C₈-Hydroxyalkylrest stehen und m gleich 1 bis 8 bedeutet,
und wobei R³ und R⁴ zusammen einen heterocyclischen Ring bilden können.

Durch den Einsatz von Feststoffkatalysatoren (Heterogenkatalysatoren) wie Zeolithe, SiO₂ mit Zeolithstruktur, Phosphate (auch Phosphate mit Zeolithstruktur), Phosphorsäure und/oder Borsäure auf Oxiden von Al, Si, Ti, Zr, Nb, Oxide der Elemente Fe, Co, Ni, Si, Al, Ti, Zr, Nb, V, Mo, W, Cr oder Gemischen der angegebenen Katalysatoren lassen sich vorteilhaft die gewünschten Alkanolamine herstellen.

Enthält das eingesetzte Epoxidgemisch noch anteilig Wasser, so entsteht durch den Einsatz von Zeolithen und Phosphaten mit Zeolithstruktur ein zusätzlicher Vorteil. Neben dem katalytischen Effekt wirken sie als Molekularsiebe und können so dem Reaktionsgemisch das Wasser entziehen. Es kann nicht zur der Bildung von zusätzlichen Diolen kommen. Die Katalysatoren wirken in diesem Fall daher auch wie Trocknungsmittel.

In den Alkanolaminen der Formel I kann es sich, je nach eingesetztem Epoxid II, bei R¹ und R² z.B. um Polymerketten aus Ethen, Propen, Isobuten oder um Styrol-Butadien-Copolymere handeln. Die Kohlenstoffzahl der Reste R¹ und R² beträgt jeweils 25 bis 350, bevorzugt 40 bis 200, insbesondere 50 bis 100. In Summe liegt die Kohlenstoffzahl der Reste R¹ und R² zwischen 25 und 350, bevorzugt 40 bis 200, insbesondere 50 bis 100, da R¹ oder R² auch Wasserstoff sein können. Bei R³ und R⁴ handelt es sich bevorzugt, je nach eingesetztem Amin bzw. NH₃, um Wasserstoff, Alkylreste mit 1 bis 10, insbesondere 1 bis 6 Kohlenstoffatomen (linear, verzweigt, z.B. Butyl, Propyl, i-Butyl), Aryl mit 6 bis 10 Kohlenstoffatomen (z.B. Phenyl), Aralkyl mit 7 bis 20 Kohlenstoffatomen (z.B. Benzyl), Alkylaryl mit 7 bis 20, insbesondere 7 bis 13 Kohlenstoffatomen (z.B. Toluyl), Aminoalkyl der oben angegebenen Formel IV (z.B. Diethylentriamin-, Triethylentetraminethylen-, Polyethylenimin-Rest), Hydroxyalkyl mit 1 bis 8 Kohlenstoffatomen (z.B. Ethanol-, Ethanolamin-, Diethanolamin-, Aminoethylethanolamin-Rest) oder cyclische Aminderivate (z.B. Morpholin-Rest). R³ kann auch eine OH-Gruppe bedeuten. Bei dem eingesetzten Amin handelt es sich dann um ein Hydroxylamin.

Als Feststoffkatalysatoren für das erfindungsgemäße Verfahren werden z.B. Zeolithe, insbesondere acide zeolithische Katalysatoren eingesetzt. Zeolithe sind kristalline Aluminosilikate, die eine hochgeordnete Struktur mit einem starren dreidimensionalen Netzwerke von SiO₄- und AlO₄-Tetraedern besitzen, die durch gemeinsame Sauerstoffatome verbunden sind. Das Verhältnis der Si- und A-Atome zu Sauerstoff beträgt 1:2 (siehe Ullmanns Encyclopädie d. techn. Chemie, 4. Auflage, Band 24, Seite 575, 1983). Die Elektrovalenz der Aluminium enthaltenden Tetraeder ist durch Einschluß von Kationen in den Kristall, z.B. eines Alkali- oder Wasserstoffions, ausgeglichen. Ein Kationenaustausch ist möglich. Die Räume zwischen den Tetraedern sind vor der Dehydration durch Trocknen bzw. Calcinieren von Wassermolekülen besetzt.

In den Zeolithen können anstelle von Aluminium auch andere Elemente wie B, Ga, Fe, Cr, Ti, V, As, Sb, Bi oder Be oder deren Gemische in das Gitter eingebaut werden, oder das Silicium kann durch ein vierwertiges Element wie Ge, Ti, Zr, Hf ersetzt werden.

Entsprechend ihrer Struktur werden Zeolithe in verschiedene Gruppen unterteilt (siehe Ullmanns Encyclopädie d. techn. Chemie, 4. Aufl., Bd. 24, S. 575, 1983). So bilden bei der Mordernit-Gruppe Ketten oder bei der Chabasit-Gruppe Schichten aus Tetraedern die Zeolith-Struktur, während sich bei der Faujasit-Gruppe die Tetraeder zu Polyedern ordnen, z.B. in Form eines Kubooktaeders, der aus Vierringen bzw. Sechsringen aufgebaut ist. Je nach Verknüpfung der Kubooktaeder, wodurch unterschiedlich große Hohlräume und Poren entstehen, unterscheidet man in Zeolithe vom Typ A, L, X oder Y.

Für das erfindungsgemäße Verfahren in Betracht kommende Katalysatoren sind Zeolithe aus der Mordernit-Gruppe oder engporige Zeolithe vom Erionit- bzw. Chabasit-Type oder Zeolithe vom Faujasit-Typ, z.B. Y-, Y- oder L-Zeolithe ebenso wie Bet-Zeolith. In diese Gruppe von Zeolithen gehören auch die sogenannten "ultrastabilen" Zeolithe des Faujasittyps, d.h. dealuminierte Zeolithe. Verfahren zur Herstellung solcher Zeolithe sind beschrieben z.B. in der US-PS 4 512 961.

Besonders vorteilhaft sind Zeolithe vom Pentasiltyp (MFI-Struktur; G.T. Kokotailo und W.M. Meier, Spec. Publ. Chem. Soc. 33 (1980) 133). Diese haben als Grundbaustein einen aus SiO₄-Tetraedern aufgebauten Fünfring gemeinsam. Sie sind durch ein hohes SiO₂/Al₂O₃-Verhältnis gekennzeichnet sowie durch Porengröße, die zwischen denen der Zeolithe vom Typ A und denen vom Typ X oder Y liegen (vgl. Ullmanns Encyclopädie d. techn. Chem., aaO).

Diese Zeolithe können unterschiedliche chemische Zusammensetzung aufweisen. Es handelt sich hierbei um Alumino-, Boro-, Eisen-, Beryllium-, Gallium-, Chrom-, Arsen-, Antimon- und Wismutsilikatzeolith oder deren Gemische sowie Alumino-, Boro-, Gallium- und Eisengermanatzeolithe oder deren Gemische oder Titansilikatzeolithe wie TS-1, ETS 4 und ETS 10.

Insbesondere eignen sich die Alumino-, Boro- Gallium- und Eisensilikatzeolithe des Pentasiltyps für das erfindungsgemäße Verfahren.

Der Aluminosilikatzeolith wird z.B. aus einer Aluminiumverbindung, vorzugsweise Al(OH)₃ oder Al₂(SO₄)₃ und einer Siliciumkomponente, vorzugsweise hochdispersem Siliciumdioxid in wäßriger Aminlösung, insbesondere in Polyaminen wie 1,6-Hexandiamin- oder 1,3-Propandiamin- oder Triethylentetramin-Lösung mit oder insbesondere ohne Alkali- oder Erdalkalizusatz bei 100 bis 220°C unter autogenem Druck hergestellt. Auch gehören hierzu die isotaktischen Zeolithe nach EP 34 727 und EP 46 504. Die erhaltenen Aluminoslikatzeolithe enthalten je nach Wahl der Einsatzstoffmengen ein SiO₂/Al₂O₃-Verhältnis von 10 bis 40 000. Auch lassen sich derartige Aluminosilikatzeolithe in etherischem Medium wie Diethylenglykoldimethylether, in alkoholischem Medium wie Methanol bzw. 1,4-Butandiol oder in Wasser synthetisieren.

Der Borosilikatzeolith wird z.B. bei 90 bis 200°C unter autogenem Druck synthetisiert, indem man eine Borverbindung, z.B. H₃BO₃, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit und insbesondere ohne Alkali- oder Erdalkalizusatz zur Reaktion bringt. Auch gehören hierzu die isotaktischen Zeolithe nach EP 34 727 und EP 46 504. Solche Borosilikatzeolithe können ebenfalls hergestellt werden, wenn man die Reaktion statt in wäßriger Aminlösung in etherischer Lösung, z.B. Diethylenglykoldimethylether oder in alkoholischer Lösung, z.B. 1,6-Hexandiol durchführt.

Der Galliumsilikatzeolith des Pentasiltyps wird z.B. bei 90 bis 200°C unter autogenem Druck synthetisiert, indem man eine Galliumverbindung, z.B. ein Alkaligallat, bevorzugt Natriumgallat, oder ein Galliumoxid oder Galliumhalogenid oder andere geeignete Galliumsalze, mit einer Siliciumverbindung, z.B. Alkalisilikaten, Kieselsolen, Kieselsäureestern oder vorzugsweise hochdispersem Siliciumdioxid in wäßriger Aminlösung, z.B. primären, sekundären oder tertiären Aminen oder quaternären Alkylammoniumverbindungen, wobei ein oder mehrere Aminfunktionen pro Molekül vorliegen können, z.B. in 1,6-Diaminohexan-Lösung oder insbesondere Tetrapropylammoniumhydroxidlösung, mit oder ohne Alkali- oder Erdalkalizusatz zur Reaktion bringt. Die Herstellung von Zeolithen in Gegenwart dieser Amine ist beispielsweise beschrieben in US-A 3 702 886 und DE-AS 30 06 471.

Den Eisensilikatzeolith erhält man z.B. aus einer Eisenverbindung, vorzugsweise Fe₂(SO₄)₃ und einer Siliciumverbindung, vorzugsweise hochdispersem Siliciumoxid in wäßriger Aminlösung, insbesondere 1,6-Hexandiamin, mit oder ohne Alkali- oder Erdalkalizusatz bei 100 bis 220°C unter autogenem Druck.

Zu den verwendbaren siliciumreichen Zeolithen (SiO₂/Al₂O₃ ≧ 10) gehören auch die sog. ZSM-Typen, Zeolith Beta, Ferrierit, EU-1, NU-1 und SiO₂ mit Zeolithstruktur (Silicalite, ein Molekularsieb, ein sog. Silica Polymorph), d.h. SiO₂-Phasen mit Pentasilstruktur, deren Kenndaten und ein Verfahren zur Herstellung, z.B. in US 4 061 724 sowie den EP-A 64 372, EP-A 93 476 und EP-A 123 060 beschrieben sind.

Auch die sogenannten ultrastabilen Zeolithe, z.B. des Faujasit-Typs oder Mordenit-Typs, das sind dealuminierte Y-Zeolithe oder dealuminierter Mordenit, deren Herstellung z.B. in der US-A 4 512 961 sowie bei H.K. Beyer und S. Belenykajy, Stud. Surf. Sc. Catal. 5 (1980) 203-209 sowie bei I.M. Newsam, Science, Vol. 231 (1986) 1094 beschrieben sind, können für das erfindungsgemäße Verfahren eingesetzt werden.

Ferner kommen Titansilikate mit Pentasilstruktur in Betracht, wie z.B. TS-1, die z.B. beschrieben werden von B. Kraushaar und I.H.C. von Haaff in Catalysis letters 1 (1988) 81-89. Ebenso können die ETS-Molekularsiebe wie z.B. ETS-4 und ETS-10 (US-A 4 853 202) eingesetzt werden.

Die Herstellung des Zeolith BETA erfolgt nach US 4 891 458 und dort nach den Beispielen 1 und 2.

Die so hergestellten Alumino-, Gallium-, Boro-, Titan- und Eisensilikatzeolithe oder Silicalite können nach ihrer Isolierung, Trocknung bei 100 bis 160°C, vorzugsweise 110°C und Calcinierung bei 450 bis 550°C, vorzugsweise 500°C, mit einem Bindemittel im Verhältnis 90:10 bis 40:60 Gew.-% zu Strängen oder Tabletten verformt werden. Als Bindemittel eignen sich diverse Aluminiumoxide, bevorzugt Boehmit, amorphe Aluminosilikate mit einem SiO₂/Al₂O₃-Verhältnis von 25:75 bis 90:5, bevorzugt 75:25, Siliciumdioxid, bevorzugt hochdispersem Al₂O₃, TiO₂, ZrO₂ sowie Ton. Nach der Verforming werden die Extrudate oder Preßlinge bei 110°C/16 h getrocknet und bei 500°C/16 h calciniert.

Man erhält auch vorteilhafte Katalysatoren, wenn die isolierten Zeolithe wie der Alumino-, Borosilikatzeolith direkt nach der Trocknung verformt wird und erst nach der Verformung einer Calcinierung unterworfen wird. Z.B. die hergestellten Alumino- und Borosilikatzeolithe können auch in reiner Form, ohne Binder, al Stränge oder Tabletten eingesetzt werden, wobei als Verstrangungs- oder Peptisierungshilfsmittel z.B. Ethylcellulose, Stearinsäure, Kartoffelstärke, Ameisensäure, Oxalsäure, Essigsäure, Salpetersäure, Ammoniak, Amine Silikoester und Graphit oder deren Gemische verwendet werden.

Liegt der Zeolith aufgrund der Art seiner Herstellung nicht in der aciden H-Form vor, sondern z.B. in der Na-Form, dann kann diese durch Ionenaustausch z.B. mit Ammoniumionen und anschließende Calcinierung oder durch Behandlung mit Säuren vollkommen oder partiell in die gewünschte H-Form überführt werden.

Wenn bei dem erfindungsgemäßen Einsatz der zeolithischen Katalysatoren eventuell eine durch Koksabscheidung bedingte Desaktivierung eintritt, empfiehlt es sich, die Zeolithe durch Abbrennen der Koksablagerung mit Luft oder mit einem Luft/N₂-Gemisch bei 400 bis 550°C, bevorzugt 500°C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Um eine möglichst hohe Selektivität, hohen Umsatz sowie lange Standzeiten zu erreichen, ist es vorteilhaft, die Zeolithe zu modifizieren. Eine geeignete Modifizierung der Katalysatoren besteht z.B. darin, daß man den unverformten oder verformten Zeolithen mit Metallsalzen durch einen Ionenaustausch oder durch Imprägnierung dotiert. Als Metalle werden Alkalimetalle wie Li, Cs, K, Erdalkalimetalle wie Mg, Ca, Sr, Metalle der 3., 4. und 5. Hauptgruppe wie Al, Ga, Ge, Sn, Pb, Bi, Übergangsmetalle der 1.-8. Nebengruppe von Ti, Zr, V, Nb, Cr, Mo, W, Mn, Re, Fe, Ru, Os, Co, Rh, Sr, Ni, Pd, Pt, Übergangsmetalle wie La, Ce, Pr, Nd, Fr, Yb und U eingesetzt.

Zweckmäßigerweise führt man die Dotierung so durch, daß man u.B. den verformten Zeolithen in einem Steigrohr vorlegt und bei 20 bis 100°C z.B. eine wäßriger ohder ammoniakalische Lösung eines Halogenids oder eines Nitrats der voranbeschriebenen Metalle überleitet. Ein derartiger Ionenaustausch kann z.B. an der Wasserstoff-, Ammonium- und Alkaliform des Zeolithen vorgenommen werden. Eine weitere Möglichkeit der Metallaufbringung auf den Zeolithen ist gegeben, indem man das zeolithische Material z.B. mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man Cu(NO₃)₂ x 3 H₂O oder Ni(NO₃)₂ x 6 H₂O oder Ce(NO₃)₃ x 6 H₂O oder La(NO₃)₂ x 6 H₂O oder Cs₂CO₃ in Wasser löst. Mit dieser Lösung wird der verformte oder unverformte Zeolith eine gewisse Zeit, ca. 30 Minuten, getränkt. Die eventuell überstehende Lösung wird am Rotationsverdampfer von Wasser befreit. Danach wird der getränkte Zeolith bei ca. 150°C getrocknet und bei ca. 550°C calciniert. Dieser Tränkvorgang kann mehrmals hinereinander vorgenommen werden, um den gewünschten Metallgehalt einzustellen.

Auch ist es möglich, z.B. eine wäßriger Ni(NO₃)₂-Lösung oder ammoniakalische Pd(NO₃)₂-Lösung herzustellen und darin den reinen pulverförmigen Zeolithen bei 40 bis 100°C unter Rühren ca. 24 h aufzuschlämmen. Nach Abfiltrieren, Trocknen bei ca. 150°C und Calcinierung bei ca. 500°C kann das so gewonnene zeolithische Material mit oder ohne Bindemittel zu Strängen, Pellets oder Wirbelgut weiterverarbeitet werden.

Ein Ionenaustausch des in der H-Form oder Ammonium-Form oder Alkali-Form vorliegenden Zeolithen kann so vorgenommen werden, daß man den Zeolithen in Strängen oder Pellets in einer Kolonne vorlegt und darüber z.B. eine wäßrige Ni(NO₃)₂-Lösung oder ammoniakalische Pd(NO₃)₂-Lösung bei leicht erhöhter Temperatur zwischen 30 und 80°C im Kreislauf 15 bis 20 h leitet. Danach wird mit Wasser ausgewaschen, bei ca. 150°C getrocknet und bei ca. 550°C calciniert. Bei manchen metalldotierten Zeolithen, z.B. Pd-, Cu-, Ni-dotierten Zeolithen, ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft. Dabei geht man vorteilhaft z.B. so vor, daß man Zeolithe in Pulverform mit 1 n Phosphorsäure 1 Stunde bei 80°C behandelt. Nach der Behandlung wird mit Wasser gewaschen, bei 110°C/16 Stunden getrocknet und bei 500°C/20 Stunden calciniert. Nach einer anderen Arbeitsweise behandelt man Zeolithe vor oder nach ihrer Verformung mit Bindemitteln, z.B. 1 bis 3 Stunden bei Temperaturen von 60 bis 80°C mit einer 3- bis 25 gew.-%igen, insbesondere 12- bis 20 gew.-%igen wäßrigen Salzsäure. Anschließend wird der so behandelte Zeolith mit Wasser gewaschen, getrocknet und bei 400°C bis 500°C calciniert.

Eine besondere Ausführungsform für die Säurebehandlung besteht darin, daß man das zeolithische Material vor seiner Verformung bei erhöhter Temperatur mit Flußsäure, die im allgemeinen als 0,001 n bis 2 n, vorzugsweise 0,05 n bis 0,5 n Flußsäure eingesetzt wird, behandelt, beispielsweise durch Erhitzen unter Rückfluß über einen Zeitraum von im allgemeinen 0,5 bis 5, vorzugsweise 1 bis 3 Stunden. Nach Isolierung, z.B. durch Abfiltrieren und Auswaschen, des zeolithischen Materials wird dieses zweckmäßig, z.B. bei Temperaturen von 100 bis 160°C, getrocknet und bei Temperaturen von im allgemeinen 450°C bis 600°C calciniert. Gemäß einer weiteren bevorzugten Ausführungsform für die Säurebehandlung wird das zeolithische Material nach einer Verformung mit Bindemittel bei erhöhter Temperatur, zweckmäßig bei Temperaturen von 50°C bis 90°C, vorzugsweise 60°C bis 80°C, über einen Zeitraum von 0,5 bis 5, vorzugsweise mit 12- bis 20 gew.-%iger Salzsäure, behandelt. Anschließend wird das zeolithische Material im allgemeinen ausgewaschen und zweckmäßig, z.B. bei Temperaturen von 100 bis 160°C, getrocknet und bei Temperaturen von im allgemeinen 450 bis 600°C calciniert. Einer HF-Behandlung kann sich auch eine HCl-Behandlung anschließen.

Nach einer anderen Arbeitsweise lassen sich Zeolithe durch Aufbringung von Phosphorverbindungen, wie Trimethoxiphosphat, Trimethoxidphosphin, primärem, sekundärem oder tertiärem Natriumphosphat modifizieren. Besonders vorteilhaft hat sich die Behandlung mit primärem Natriumphosphat erwiesen. Hierbei werden die Zeolithe in Strang-, Tabletten- oder Wirbelgut-Form mit wäßriger NaH₂PO₄-Lösung getränkt, bei 110°C getrocknet und bei 500°C calciniert.

Weitere Katalysatoren für das erfindungsgemäße Verfahren sind Phosphate, insbesondere Aluminiumphosphat, Siliciumaluminiumphosphate, Cerphosphat, Zirkonphosphate, Borphosphat, Eisenphosphat oder deren Gemische.

Als Aluminiumphosphat- und siliciumaluminiumphosphat-Katalysatoren werden für das erfindungsgemäße Verfahren insbesondere unter hydrothermalen Bedingungen synthetisierte substituierte und unsubstituierte Aluminiumphosphate und Siliciumaluminiumphosphate eingesetzt.

Die den Zeolithen strukturverwandten Aluminiumphosphatkatalysatoren werden für das erfindungsgemäße Verfahren insbesondere unter hydrothermalen Bedingungen synthetisiert. Es sind Siliciumaluminophsphate (Acronym SAPO) oder Aluminiumphosphate (Acronym AlPO₄). Diese kristallinen Festkörper weisen definierte Hohlraum- und Porenstrukturen auf und sind mit den Zeolithen strukturverwandt.

Die Herstellung, Eigenschaften und die Klassifizierung dieser Festkörper auf der Basis von Struktur und chemischer Zusammensetzung werden ausführlich beschrieben in Pure and App. Chem., Vol. 58, No. 10, pp. 1317-22 und pp. 1351-58 (1986).

Aus den derzeit bekannten Kristallstrukturen und den zahlreichen Elementmodifizierungen sind inzwischen gut 700 verschiedene Kombinationen möglich. Die Bezeichnung der Aluminophosphate erfolgt durch folgende Acronyme AlPO₄, SAPO, MeAPO, MeAPSO, ElAPO oder ElAPSO. Dabei bedeuten A oder Al Aluminium, S Silicium, P steht für Phosphor und O für Sauerstoff. Unter Me sind Metalle von Fe, Mg, Mn, Co und ZN und unter El Elemente wie Be, Ga, Ge, Ti, As, B oder Li zu verstehen. Eine mit Bindestrich angefügte Zahl dient der Kennzeichnung der Kristallstruktur der betreffenden Phase.

Die unter hydrothermalen Bedingungen hergestellten Aluminiumphosphate sind z.B. AlPO-5, AlPO-9, AlPO-11, AlPO-12, AlPO-14, AlPO-21, AlPO-25, AlPO-31, AlPO-33, AlPO-34, AlPO-37 und AlPO-54. Synthesen dieser Verbindungen sind in EP 132 709, US 4 310 440 und US 4 473 663 beschrieben.

Beispielsweise das AlPO₄-5 (APO-5) wird synthetisiert, indem man Orthophosphorsäure mit Pseudoboehmit (Catapal SB®) in Wasser homogen mischt; zu dieser Mischung Tetrapropylammoniumhydroxid gibt und danach bei ca. 150°C 20 bis 60 h unter autogenem Druck in einem Autoklaven umsetzt. Das abfiltrierte AlPO₄ wird getrocknet bei 100 bis 160°C und calciniert bei 450 bis 550°C.

AlPO₄-9 (APO-9) wird ebenfalls aus Orthophosphorsäure und Pseudoboehmit aber in wäßriger DABCO-Lösung (1,4-Diazabicyclo-(2,2,2)octan) bei ca. 200°C unter autogenem Druck während 200 bis 400 h synthetisiert.

Die Synthese des AlPO₄-21 (APO-21) erfolgt aus Orthophosphorsäure und Pseudoboehmit in wäßriger Pyrrolidon-Lösung bei 150 bis 200°C unter autogenem Druck während 50 bis 200 h.

Die Synthese für MeAPO's sind US-A 4 544 143, EP-A 132 708 und US-A 4 567 029 sowie für ElAPO in US-A 4 500 651 und EP-A 2158 976 beschrieben.

Für das erfindungsgemäße Verfahren bevorzugt sind insbesondere die Silicium-haltigen Aluminophosphate (SAPO, MeAPSO oder ElAPSO) wie SAPO-11, SAPO-5, SAPO-20, SAPO-34, SAPO-37, SAPO-41 oder SAPO-46. Synthesen der Silicium-aluminophosphate sind u.a. beschrieben für SAPO in US-A 4 440 871 und EP-A 103 117, für MeAPSO in EP-A 158 348, EP-A 158 975 und EP-A 161 491, und für ElAPSO in EP-A 159 624.

SAPO's werden hergestellt durch Kristallisation aus wäßriger Mischung bei 100 is 250°C und autogenem Druck während 2 h bis 2 Wochen, wobei die Reaktionsmischung aus einer Silicium-, Aluminium- und Phosphorkomponente in wäßrigen aminoorganischen Lösungen umgesetzt wird.

Als Siliciumaluminiumphosphate sind z.B. ZYT-5, ZYT-6, ZYT-7, ZYT-9, ZYT-11 und ZYT-12 geeignet (J 5 9217-619).

SAPO-5 beispielsweise wird durch Mischen von SiO₂ suspendiert in wäßriger Tetrapropylammoniumhydroxid-Lösung mit einer wäßrigen Suspension aus Pseudoboehmit und Orthophosphorsäure und anschließende Umsetzung bei 150 bis 200°C während 20 bis 200 h unter autogenem Druck in einem Rührautoklaven erhalten. Die Trocknung des abfiltrierten Pulvers erfolgt bei 110 bis 160°C und die Calcination bei 450 bis 550°C.

Als Phosphatkatalysatoren kann man bei dem Verfahren auch gefällte Aluminiumphosphate einsetzen. Beispielsweise wird ein derartiges Aluminiumphosphat hergestellt, indem 92 g Diammoniumhydrogenphosphat in 700 ml Wasser gelöst werden. Zu dieser Lösung wird 260 g Al(NO₃)₃ x H₂O in 700 ml Wasser über 2 h zugetropft. Dabei wird der pH-Wert durch gleichzeitige Zugabe von 25 %iger NH₃-Lösung bei pH 8 gehalten. Der entstandene Niederschlag wird 12 h nachgerührt, dann abgesaugt und ausgewaschen. Er wird bei 60°C/16 h getrocknet.

Borphosphate für das erfindungsgemäße Verfahren lassen sich beispielsweise durch Mischen und Kneten von konzentrierter Borsäure und Phosphorsäure und durch anschließende Trocknung und Calcination in Inertgas-, Luft- oder Dampfatmosphäre bei 250 bis 650°C, vorzugsweise 300 bis 500°C herstellen.

CePO₄ wird durch Fällung aus 52 g Ce(NO₃)₃ x 6 H₂O und 56 g NaH₂PO₄ x 6 H₂O erhalten. Nach der Filtration wird das Material zu Strängen verformt, bei 120°C getrocknet und bei 450°C calciniert. Der Katalysator enthält 47,1 Gew.-% Ce und 12,7 Gew.-% P.

Als Zirkonphosphate kommen im Handel erhältliche Zirkonphosphate wie z.B. CSZ 100, Zirkonphosphatsilikate und Zirkonphosphate, die NH₃ adsorbieren bzw. adsorbiert haben, in Betracht.

Phosphorsäure wird auf SiO₂-, Al₂O₃-, TiO₂, ZrO₂, Nb₂O₅ oder Bims-Träger z.B. durch Auftränken oder Versprühen aufgebracht. Ein Phosphorsäure-haltiger Katalysator kann beispielsweise durch Auftränken von H₃PO₄- oder NaH₂HPO₄- oder Na₂HPO₄-Lösung auf SiO₂ und anschließende Trocknung bzw. Calcination erhalten werden. Phosphorsäure kann aber auch mit Kieselgel zusammen in einem Sprühturm versprüht werden, danach schließt sich eine Trocknung und meist eine Calcination an. Phosphorsäure kann auch in einer Imprägniermühle auf das Trägermaterial aufgesprüht werden.

Geeignete unbehandelte Katalysatoren sind z.B. auch die Metalloxide, insbesondere sauer wirkenden Oxide der Elemente Ti, Zr, Si, Al, Fe, Co, Ni, V, W, Mo, Nb und Cr. Es sind dies Titandioxid, Zirkondioxid, Vanadiumoxide, Nioboxide, Chromoxide, Molybdänoxide, Wolframoxide etc. oder Gemische dieser Oxide. So kann man nach dem erfindungsgemäßen Verfahren mit diesen Katalysatoren ebenfalls die gewünschten Produkte herstellen.

Die hier beschriebenen Katalysatoren können wahlweise als 2- bis 4 mm-Stränge oder als Tabletten mit 3 bis 5 mm Durchmesser oder als Splitt mit Teilchengrößen von 0,1 bis 0,5 mm oder als Wirbelgut eingesetzt werden. Sie haben als Feststoffkatalysatoren den zusätzlichen Vorteil, daß sie einfach aus dem Reaktionsgemisch abgetrennt und wiederverwendet werden können.

Die bevorzugt eingesetzten Polyisobutene haben ein Molekulargewicht von etwa 400 bis 5000, vorzugsweise von 800 bis 1500. Sie werden in bekannten Verfahren durch kationische Polymerisation von Isobuten erhalten.

Die Herstellung der Epoxide II erfolgt mittels bekannter Epoxidierungsreagentien (s. DE 40 30 164).

Die für die erfindungsgemäße Umwandlung in der Regel gewählten Reaktionstemperaturen liegen bei 20°C bis 600°C, bevorzugt bei 50 bis 500°C, inbesondere bei 100 bis 350°C.

Die Reaktionszeiten können von 30 min bis zu 100 Stunden betragen.

Bevorzugt ist es, die Reaktion in der Flüssigphase, insbesondere in Suspensions-, Riesel- oder Sumpffahrweise, bei Temperaturen von 50 bis 300°C, bevorzugt von 100 bis 250°C und einer Belastung WHSV (weight hour space velocity) = 100 h-¹ bis 0,5 h⁻¹, bevorzugt 60 h-¹ bis 10 h⁻¹ durchzuführen.

Als Lösungsmittel sind generell inerte Lösungsmittel einsetzbar, z.B. Cyclohexan, Toluol oder Petrolether. Der Reaktions-Autoklav besteht z.B. aus nicht korrodierendem Edelstahl. Die Menge des eingesetzten Katalysators beträgt allgemein 0,01 bis 20 Gew.-%, bezogen auf das eingesetzte Epoxid.

Das Verfahren wird in der Regel bei Normaldruck oder erhöhtem Druck, d.h. bei 1 bis 800 bar, bevorzugt 50 bis 500 bar, insbesondere 150 bis 350 bar durchgeführt. Es kann kontinuierlich oder diskontinuierlich durchgeführt werden.

Schwerflüchtige oder feste Edukte werden in gelöster Form, z.B. in THF-, Toluol-, Cyclohexan- oder Petroether-Lösung zum Einsatz gebracht. Generell ist eine Verdünnung des Eduktgemischs mit derartigen Lösungsmitteln oder mit Inertgas wie N₂ oder Ar möglich.

Die folgenden Beispiele veranschaulichen die Erfindung.

### Herstellung der Alkanolamine

### Beispiele 1 bis 25

Die Reaktionen in der Flüssigphase wurden unter isothermen Bedingungen in einem HC-Autoklaven (300 ml) durchgeführt. Im einzelnen wurde dabei wie folgt vorgegangen: 1 Volumenteil (50 g) Polyisobutenepoxid (Molmasse ca. 1000) wurde in 50 ml Cyclohexan bei Raumtemperatur gelöst und 1 g Feststoffkatalysator (siehe Tabelle I) zugegeben; dann wurde die aus der Tabelle I ersichtliche Menge NH₃ aufgepreßt und die Reaktionsmischung wurde unter autogenen Bedingungen auf die ebenfalls in der Tabelle I angegebene Temperatur gebracht. Die sich einstellenden Drücke sowie die Reaktionszeiten und Ausbeuten an Alkanolaminen (GlAA) sind auch aus der Tabelle I ersichtlich. Die Reaktionsprodukte wurden durch übliche Kennzahlenbestimmungen charakterisiert (Hydrierjodzahl nach DIN 53 241, Teil 2 oder Ind. Chim. Belge 32, 134 (1967); Aminzahl nach DIN 16 945 oder DAB9 (s. auch ASTM D 2073-66); Epoxide nach DIN 16 945 oder DAB9, vgl. auch Hofmann und Stark: The Determination of Epoxide Groups, Pergamon Press, Oxford, London, 1969; Hydroxylzahl nach DIN 16 945 oder DAB9, siehe auch DIN 53 240 oder ASTM D 2849-69).

**Tabelle I**

| Beispiel 1-25 | | | | | | |
|---|---|---|---|---|---|---|
| Vers. -Nr. | Kat. | Verhältnis Epoxid/NH₃ | Temp. [°C] | Druck [bar] | Zeit [h] | Ausbeute [%] GlAA bez. auf Epoxid |
| 1 | A | 1/4 | 150 | 137 | 60 | 46 |
| 2 | A | 1/4 | 200 | 300 | 60 | 76 |
| 3 | A | 1/4 | 250 | 402 | 60 | 62 |
| 4 | B | 1/2 | 50 | 20 | 20 | 31 |
| 5 | B | 1/2 | 100 | 56 | 20 | 31 |
| 6 | B | 1/2 | 150 | 110 | 20 | 35 |
| 7 | B | 1/2 | 200 | 155 | 20 | 52 |
| 8 | C | 1/2 | 100 | 56 | 20 | 31 |
| 9 | C | 1/2 | 150 | 110 | 20 | 45 |
| 10 | C | 1/2 | 200 | 155 | 20 | 53 |
| 11 | D | 1/2 | 50 | 14 | 21 | 17 |
| 12 | D | 1/4 | 200 | 270 | 21 | 47 |
| 13 | E | 1/2 | 50 | 15 | 20 | 21 |
| 14 | E | 1/4 | 200 | 270 | 60 | 59 |
| 15 | F | 1/2 | 50 | 10 | 20 | 25 |
| 16 | F | 1/4 | 200 | 290 | 60 | 59 |
| 17 | G | 1/2 | 50 | 17 | 20 | 19 |
| 18 | G | 1/4 | 200 | 270 | 21 | 73 |
| 19 | H | 1/6 | 200 | 584 | 60 | 59 |
| 20 | I | 1/2 | 50 | 20 | 20 | 38 |
| 21 | I | 1/2 | 100 | 56 | 20 | 41 |
| 22 | I | 1/2 | 150 | 110 | 20 | 41 |
| 23 | I | 1/2 | 200 | 155 | 20 | 50 |
| 24 | J | 1/4 | 200 | 238 | 80 | 57 |
| 25 | K | 1/6 | 190 | 680 | 60 | 50 |

### Beispiel 26 - 28

Die Reaktionen wurden in der Flüssigphase unter isothermen Bedingungen im Rührkessel (250 ml) analog den Beispielen 1-25 durchgeführt. Die Reaktionsprodukte wurden durch die genannten Kennzahlenbestimmungen charakterisiert. Umgesetzt wird ein Polyisobutenepoxid (Molmasse ca. 1000) mit Morpholin zu dem entsprechenden Alkoholamin (GlAAm) (s. Tabelle II)

**Tabelle II**

| Vers.-Nr. | Kat. | Verhältnis Epoxid/Morpholin | Temp. [°C] | Druck [bar] | Zeit [h] | Ausbeute [%] GlAAm* |
|---|---|---|---|---|---|---|
| 26 | A | 1/4 | 102 | 1 | 10 | 14.4 |
| 27 | A | 1/4 | 102 | 1 | 60 | 57,2 |
| 28 | A | 1/4 | 102 | 1 | 72 | 71.5 |

| | | | | | | |
|---|---|---|---|---|---|---|
| * bezogen auf eingesetztes Epoxid | | | | | | |

Die in den Beispielen eingesetzten Katalysatoren wurden wie folgt hergestellt:

### Katalysator A

Ein Aluminosilikatzeolith vom Pentasil-Typ wurde unter hydrothermalen Bedingungen bei autogenem Druck und 150 °C aus 65 g hochdispersem SiO₂, 203 g Al₂(SO₄)₃ x 18 H₂O in 10 kg einer wäßrigen 1,6-Hexandiamin-Lösung (Mischung 50:50 Gew.-%) in einem Rührautoklaven hergestellt. Nach Abfiltrieren und Auswaschen wurde das kristalline Reaktionsprodukt bei 110 °C/24 h getrocknet und bei 500 °C/24 h kalziniert. Dieser Aluminosilikatzeolith enthielt 92,8 Gew.-% SiO₂ und 4,2 Gew.-% Al₂O₃. Mit diesem Material werden durch Verformen mit einem Verformungshilfsmittel 2-mm-Stränge hergestellt, die bei 110 °C/16 h getrocknet und bei 500 °C/24 h kalziniert werden.

### Katalysator B

Katalysator B wurde hergestellt, indem ein handelsüblicher Na-Y-Zeolith zu 2-mm-Strängen verformt wurde, die bei 110 °C/16 h getrocknet und bei 500 °C/16 h kalziniert wurden.

Die Stränge wurden mit 20%iger NH₄Cl-Lösung (Massenverhältnis 1:15) bei 80 °C ausgetauscht. Dann wurde Chlorid-frei gewaschen, bei 110 °C getrocknet und 5 h bei 500 °C kalziniert. Der Na-Gehalt betrug 0,07 %.

### Katalysator C

Katalysator C wurde hergestellt, indem ein handelsüblicher Na-Y-Zeolith in der Pulverform mit pyrogener Kieselsäure (Gewichtsverhältnis 80:20) zu 2-mm-Strängen verformt wurde, die bei 110 °C/16 h getrocknet und bei 500 °C/16 h kalziniert wurden.

Die Stränge wurden mit 20%iger NH₄Cl-Lösung (Massenverhältnis 1:15) bei 80 °C ausgetauscht. Dann wurde Chlorid-frei gewaschen, bei 110 °C getrocknet und 5 h bei 500 °C kalziniert. Der Na-Gehalt betrug 0,07 %.

### Katalysator D

Katalysator D wurde hergestellt, indem man Pseudoboehmit mit 2 % HCOOH zu 2-mm-Strängen verformte, bei 110 °C/16 h trocknete und bei 500 °C/16 h kalzinierte.

### Katalysator E

Handelsübliches TiO₂ (Fa. Degussa)

### Katalysator F

Handelsübliches ZrO₂ (Fa. Mel)

### Katalysator G

α-Al₂O₃ (BASF-Katalysator K 10)

### Katalysator H

Katalysator H wurde erhalten, indem man die Stränge des Katalysators A mit 0,1 n HF 1 h behandelte, nach dem Filtrieren neutral wusch, bei 130 °C/2 h trocknete und bei 540 °C/2 h kalzinierte.

### Katalysator I

Katalysator I wurde hergestellt, indem man Katalysator A mit pyrogener Kieselsäure (Gewichtsverhältnis (80:20)) zu 2-mm-Strängen verformte, die bei 110 °C/16 h getrocknet und bei 500 °C/16 h kalziniert wurden.

Die Stränge wurden mit 20%iger NH₄Cl-Lösung (Massenverhältnis 1:15) bei 80 °C ausgetauscht. Dann wurde Chlorid-frei gewaschen, bei 110 °C getrocknet und 5 h bei 500 °C kalziniert.

### Katalysator J

Handelsüblicher ZSM-5 Zeolith (CBM 3020, Fa. Conteka).

### Katalysator K

Katalysator K wurde hergestellt, indem Katalysator H mit pyrogener Kieselsäure zu 2-mm-Strängen verformt wurde, die bei 110 °C/16 h getrocknet und bei 500 °C/16 h kalziniert wurden.

Die Stränge wurden mit 20%iger NH₄Cl-Lösung (Massenverhältnis 1:15) bei 80 °C ausgetauscht. Dann wurde Chlorid-frei gewaschen, bei 110 °C getrocknet und 5 h bei 500 °C kalziniert.

### Anwendungstechnische Versuche

Das jeweilige Additiv wurde mit einem Anteil von 800 ppm dem Kraftstoff zugefügt. Der Test wurde auf jeweils zwei Ventilen eines Opel Kadett-Motors nach Norm CEC-F-02-T-79 durchgeführt. Die Beurteilung der Additivqualität erfolgte durch Auswiegen der Ventilstößel. Die Gewichtszunahme entspricht der "Coke"-Abscheidung am Ventileingang. Die Ergebnisse sind aus Tabelle III ersichtlich.

**Tabelle III**

| | Gewichtszunahme (mg) | |
|---|---|---|
| | Ventil 1 | Ventil 2 |
| Kraftstoff-Grundwert | 440 | 564 |
| Additiv aus Vers. 3 | 86 | 15 |
| Additiv aus vers. 1 | 35 | 15 |
| Additiv aus vers. 18 | 13 | 35 |

## Patentansprüche

1. Verfahren zur Herstellung von Alkanolaminen der Formel I, 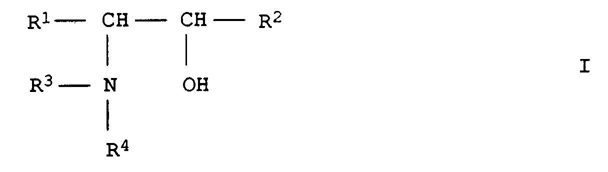 worin bedeuten
R¹ und R² unabhängig voneinander Wasserstoff oder einen, gegebenenfalls Aryl-substituierten, gesättigten oder ungesättigten aliphatischen Rest mit 25 bis 350 C-Atomen, mit der Maßgabe, daß zumindest einer der beiden Reste R¹ und R² der genannte Alkylrest ist und die Summe der C-Atome in den Resten R¹ und R² 25 bis 350 beträgt,
und
R³ eine OH-Gruppe
sowie
R³ und R⁴ unabhängig voneinander Wasserstoff oder Alkyl-, Hydroxyalkyl-, Aryl-, Aralkyl-, Alkaryl- oder Aminoalkylreste, die noch durch weitere Hydroxy- oder Aminogruppen tragende Alkylreste substituiert sein können, wobei R³ und R⁴ zusammen einen heterocyclischen Ring bilden können, dadurch gekennzeichnet, daß man Epoxide der Formel II 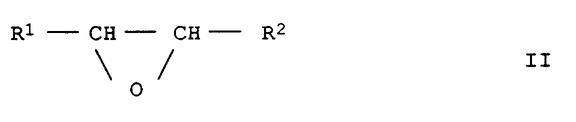 in Gegenwart von Feststoffkatalysatoren mit NH₃ oder Aminen der Formel III 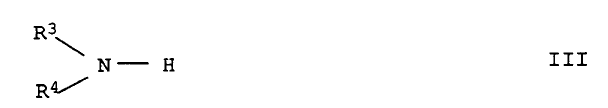 umsetzt, wobei R¹, R², R³ und R⁴ die oben genannten Bedeutungen aufweisen.

2. Verfahren nach Anspruch 1, dadurch gekennzeichnet, daß man als Katalysatoren Zeolithe, SiO₂ mit Zeolithstruktur, Phosphate, Phosphorsäure und/oder Borsäure auf Oxiden von Al, Si, Ti, Zr, Nb, Oxide der Elemente Fe, Co, Ni, Si, Al, Ti, Zr, Nb, V, Mo, W, Cr oder Gemische der angegebenen Katalysatoren einsetzt.

3. Verfahren nach Anspruch 1, dadurch gekennzeichnet, daß R³ und R⁴ unabhängig voneinander bedeuten:
Wasserstoff, einen C₁-C₁₀-Alkylrest, einen C₆-C₁₀-Arylrest, einen C₇-C₂₀-Aralkylrest, einen C₁-C₈-Hydroxyalkylrest, einen C₇-C₂₀-Alkylarylrest oder einen Aminoalkylrest der Formel IV
⁅R⁵―NR⁶⁆ₘR⁷ IV
worin R⁵ für einen C₂-C₅-Alkylenrest und R⁶ sowie R⁷, die gleich oder verschieden sind, für Wasserstoff, einen C₁-C₆-Alkylrest, C₆-C₁₀-Arylrest oder C₁-C₈-Hydroxyalkylrest stehen und m gleich 1 bis 8 bedeutet,
und wobei R³ und R⁴ zusammen einen heterocyclischen Ring bilden können.

4. Verfahren nach Anspruch 1, dadurch gekennzeichnet, daß man als Epoxid (III) Polyisobutylepoxid als Ausgangsstoff einsetzt.

5. Verfahren nach Anspruch 2, dadurch gekennzeichnet, daß man als Feststoffkatalysatoren Zeolithe des Pentasil-Typs verwendet.

6. Verfahren nach Anspruch 2, dadurch gekennzeichnet, daß man mit Übergangsmetallen, Erdmetallen und Alkalimetallen dotierte Feststoffkatalysatoren verwendet.

7. Verfahren nach Anspruch 2, dadurch gekennzeichnet, daß man mit Säuren behandelte Feststoffkatalysatoren verwendet.

8. Verfahren nach Anspruch 1, dadurch gekennzeichnet, daß man die Reaktion bei 20°C bis 600°C und bei Drücken von 1 - 800 bar, insbesondere in der Flüssigphase, durchführt.

9. Verwendung der nach dem Verfahren gemäß Anspruch 1 hergestellten und von dem Feststoffkatalysator abgetrennten Reaktionsmischung als Kraftstoff- oder Schmierstoffadditiv.

10. Verwendung nach Anspruch 9, dadurch gekennzeichnet, daß die Reaktionsmischung, gegebenenfalls nach Abtrennung von Lösungsmitteln, dem Kraftstoff in einer Menge von 10 - 5000 ppm oder dem Schmierstoff in einer Menge von 0,1 bis 6 Gew.-% als Additiv zugegeben wird.
